# EUROPEAN PATENT APPLICATION

(11) **EP 3 050 959 A1**
(43) Date of publication of application: **03.08.2016**
(21) Application number: 14848820.8
(22) Date of filing: 19.09.2014
(51) Int. Cl.: C12M 1/34, G01N 33/48

(54) **CELL-OBSERVATION-INFORMATION PROCESSING SYSTEM, CELL-OBSERVATION-INFORMATION PROCESSING METHOD, CELL-OBSERVATION-INFORMATION PROCESSING PROGRAM, RECORDING UNIT FOR CELL-OBSERVATION-INFORMATION PROCESSING SYSTEM, AND DEVICE FOR CELL-OBSERVATION-INFORMATION PROCESSING SYSTEM**

(30) Priority: 26.09.2013 JP 2013200189
(71) Applicant: Olympus Corporation, Shibuya-ku Tokyo 151-0072 (JP)
(72) Inventor: IGA Yasunobu, Tokyo 151-0072 (JP); TANIKAWA Yohei, Tokyo 151-0072 (JP); TAKIMOTO Shinichi, Tokyo 151-0072 (JP)
(74) Representative: Gunzelmann, Rainer
(86) International application number: PCT/JP2014/074838
(87) International publication number: WO 2015/046052

(57) **Abstract**

[Object] To provide a cell observation information processing system that enables users to efficiently conduct condition management of cells used for research of living object.

[Measures for solution] Including a tag acquiring section 11 that acquires, as search tags for searching for observation information acquired by an observation information acquiring section 20 in response to an acquisition order at one time point, user identifying information, cell identifying information, information on date and time of observation information acquisition, information on maintenance work, apparatus identifying information, activity data variation information, and an image tag; a storing section 12 that stores, into an archive section 13, respective sets of the observation information acquired at respective time points upon assigning to them the search tags; a search criterion acquiring section 14 that acquires, as a search criterion, information containing at least one of the information items and the image tag that constitute the search tags; and a retrieving section 15 that searches through the archive section 13 using the search criterion as acquired and retrieves data containing search-tagged observation information to which search tags that are matched with the search criterion have been assigned.

## Description

### TECHNICAL FIELD

The present invention relates to a cell observation information processing system, a cell observation information processing method, a cell observation information processing program, an archive section provided for the cell observation information processing system, and apparatuses provided for the cell observation information processing system, each of which is to be used for condition management of cells to be used for research of living object upon use of observation information on the cells acquired by an observation information acquiring apparatus such as a microscope.

### BACKGROUND ART

In the fields where experiment and research using cells are to be made, users continue culturing cells on a daily basis and control, through observation of cell images via microscopes, conditions of the cells to be used for the experiment and research.

In general, cells to be used for experiment and research are separated from the living body and cultured upon being planted in a culture medium in a certain culture container, to be proliferated. These cells to be cultured are called parent cell line. When cells in a parent cell line are proliferated to a predetermined confluency or number, a part of the cells in the culture container of the parent cell line are transplanted to a culture medium in another culture container, to be cultured and proliferated there. This process is called subculture (passage of culture). The cells for experiment and research separated from the parent cell line are called cells for experiment.

In experiment and research using cells, users repeat passage of culture for proliferating and maintaining the parent cell line, and use cells in a container of cells for experiment separated from the subcultured parent cell line.

Therefore, to improve accuracy of the experiment and research, it is desired that cells used for each time of experiment and research are kept in good culture conditions.

In the course of culturing cells, the cell conditions may be varied.

Variation of the cell conditions includes short-term variation, such as abnormality conspicuous at a single glance like contamination, and long-term variation of the order of several weeks. Regarding the long-term variation, it is difficult to recognize it only through a cell image acquired at one time point.

Conventionally, cell conditions have been recognized by eye observation, through microscopes, of cells in culture containers by users. Finding variations of the cell conditions has been entrusted to memory and sense of users. However, entrusting it to the users' memory and sense would hinder accurate recognition of a long-term variation of the cell conditions, to lead to failure in enhancing accuracy of experiment and research. For accurate recognition of long-term variations of cell conditions, it is necessary to make observation information at individual time points searchable by compiling it into a database.

Conventionally, there has been proposed an image processing apparatus that has an image database in which and through which observation data can be stored and searched, in the following Patent Document 1, for example.

The apparatus described in Patent Document 1 is configured so that it makes it possible to store, in a certain relational database, observation images containing images of living specimens, upon assigning to them information on acquisition conditions at the times of capturing the observation images, such as photographing conditions and photographing date/time, to designate the information on acquisition conditions as a search criterion, and to choose and display images that are matched with the search criterion.

### PRIOR ART DOCUMENTS

### PATENT DOCUMENTS

Patent Document 1: US Patent Appln. Pub. No. 2006/0206482

### SUMMARY OF THE INVENTION

### PROBLEMS TO BE SOLVED BY THE INVENTION

However, Patent Document 1 merely discloses the configuration for storing, in a database, images and acquisition conditions upon linking them to each other. The invention described in Patent Document 1 has no conception of using the database for cell culturing or passage culturing. Therefore, when the apparatus described in Patent Document 1 is applied to management of cells that are used for experiments, there exist the problems shown below in which the circumstances peculiar to the field of experiment and research using cells make it difficult to isolate, as retrieved data, data of the past regarding the cells under use.

### Problem 1 (resulted from the circumstance that the manner of cell management differs by user)

In experiments using cells, there is a custom or tendency that users individually manage cells they use. Therefore, in the apparatus described in Patent Document 1, in a case where a plurality of users store observation data of cells and conducts searches using the same database, not only observation data of cells managed by the user in charge of the experiment but also observation data of cells managed by other users are retrieved altogether. As a result, it is rendered bothersome or difficult to isolate, as retrieved data, desired observation data on cells under the management of the very experimenter.

That is, in observation and experiment of cells, in accordance with the conventional custom, cell management tends to be operated not such that a plurality of users conduct cell management upon sharing the same cells but such that a particular limited user, namely, the researcher himself/herself or a skilled cell-management technician who is entrusted with the cell-management work for the researcher manages the cells throughout their entire lifecycle from birth to killing. This is because replacement of the user in the midst of managing the cells would lead to variation in characteristics of the cells caused by difference in user-specific cell management manner between individuals, which then would lead to an observation result with degraded accuracy such as improper chronological variation regarding activity data such as cell confluency, which indicates conditions of the cultured cells.

The apparatus described in Patent Document 1 is not a system designed in consideration of the above-described peculiar custom in experiments using cells, namely, that cells used for cell-using experiments should be managed by individual users. In the first place, Patent Document 1 is not configured for use by a plurality of users and thus has no function of searching data on an individual user basis. Therefore, under the situation where a plurality of users use the same database in the apparatus described in Patent Document 1, even if each user wishes to grasp only conditions of the cells under his or her own management, the search result would contain, also, observation data on cells under management by others, which are, for the user, unnecessary data not worth referring to. As a result, the work of condition check on the cells under management by the user would be rendered bothersome and take much time. In particular, in the situation where the condition check on the cells is conducted while the cells are taken outside the incubator, such as in the midst of passage work, the condition check on the cells, if taking much time, would seriously damage the cells.

### Problem 2 (chronological retrieval of data group on cell basis cannot be made)

The apparatus described in Patent Document 1 does not allow a data group acquired over a plurality of time points of the past to be retrieved on a cell basis in bulk.

As stated above, variation of the cell conditions includes short-term variation, such as abnormality surely recognized by observation of a cell image acquired at one time point, like contamination, and long-term variation, which cannot be recognized unless the chronological variation of images of identical cells acquired over a plurality of time points is checked. Therefore, it is necessary to designate search criterion that facilitates retrieval of observation results of the identical cells acquired over a plurality of different time points.

In general, when cells are to be subcultured, cells for formal experiment are separated and taken out from cells having been cultured in a culture container of a parent cell line, and the cells from which the separation is made (parent cell line) and the taken-out cells (cells for experiment) are given the same cell name which is distinguishable from cell names given by other users.

However, since the apparatus described in Patent Document 1 is not configured to allow a data group acquired over a plurality of time points of the past to be retrieved on a cell basis in bulk, a long-term variation of cell conditions cannot be recognized.

### Problem 3 (search using information on user's work for cell maintenance is not considered)

In the apparatus described in Patent Document 1, search using information on user's work for cell maintenance is not taken into consideration.

For example, a user basically checks cell conditions every few days. By checking every few days, the user can grasp the macro variation of the cell conditions. Regarding the cell conditions inside the culture container, when the user confirms that the activity of the cells has reached a predetermined level in terms of cell confluency, number of cells or so, he or she conducts maintenance work for passage.

On the other hand, the user desires to check, using activity data of the cells, daily cell conditions during a period in which the activity has not yet reached the level as requiring passage of culture.

If the configuration is made so that the observation data required for maintenance work for passage of culture, which are checked every few days, and the daily observation data during the period in which the activity has not yet reached the level as requiring passage of culture are separately retrievable, such configuration can preclude unnecessary observation data from being retrieved, to shorten the work time, and thus is desirable.

However, in the apparatus described in Patent Document 1, since a search using information on user's work for cell maintenance is not taken into consideration, unnecessary observation data on other works cannot be precluded, and thus the work time cannot be shortened.

### Problem 4 (no function is provided for enabling search on the basis of apparatus via which observation data on cells have been acquired)

The apparatus described in Patent Document 1 is not provided with a function for enabling search on the basis of apparatus via which observation data on cells have been acquired. In the first place, it is not configured on the assumption that observation data on cells acquired by a plurality of apparatuses should be recorded.

For example, if a formal experiment fails, it is necessary to check images of the past retrospectively for recognizing the cell conditions.

In a system configuration in which users acquire observation images using a plurality of apparatuses and record observation results, image capture situations differ by apparatus. Therefore, in the event of checking cell conditions on the basis of past observation images of the cells, it is desirable that the image capture situations specific to individual apparatuses, via which the observation images of the cells have been acquired, are taken into consideration.

For example, the lamp of some apparatus approaching the end of its lifetime has degraded image quality, malfunction of the laser of some apparatus has damaged the cells, etc.-such image capture situations are specific to individual apparatuses. Therefore, a search on the apparatus basis would facilitate smooth understanding of cause of the failure.

However, since the apparatus described in Patent Document 1 is not provided with a function of search on the basis of apparatus via which observation images of cells are to be acquired, it is difficult to smoothly understand the cause of the failure.

### Problem 5 ( search cannot be conducted on the basis of variation of activity data)

According to the apparatus described in Patent Document 1, search cannot be conducted on the basis of variation of activity data, which indicate activity of cells in terms of cell confluency, survival rate etc.

To enhance the success rate of a formal experiment, it is necessary to choose vigorous cells having as high activity as possible.

In addition, to enhance repeatability of a formal experiment, it is desirable to choose cells having the same level of activity as those used in the previous formal experiment.

However, in the apparatus described in Patent Document 1, a search cannot be conducted on the basis of variation of activity data, and thus it is difficult to enhance success rate and repeatability of a formal experiment.

A few aspects of the present invention are proposed to solve the above-described conventional problems; an object of the present invention is to provide a cell observation information processing system, a cell observation information processing method, a cell observation information processing program, an archive section provided for the cell observation information processing system, and an apparatus provided for the cell observation information processing system, each of which enables users to efficiently conduct condition management of cells used for research of living object.

Alternatively, according to a few aspects, an object of the present invention is to provide a cell observation information processing system, a cell observation information processing method, a cell observation information processing program, an archive section provided for the cell observation information processing system, and an apparatus provided for the cell observation information processing system, each of which enables users to conduct, in a short time, condition management of cells used for research of living object.

Alternatively, according to a few aspects, an object of the present invention is to provide a cell observation information processing system, a cell observation information processing method, a cell observation information processing program, an archive section provided for the cell observation information processing system, and an apparatus provided for the cell observation information processing system, each of which enables users to accurately conduct condition management of cells used for research of living body.

Alternatively, according to a few aspects, an object of the present invention is to provide a cell observation information processing system, a cell observation information processing method, a cell observation information processing program, an archive section provided for the cell observation information processing system, and an apparatus provided for the cell observation information processing system, each of which enables users to rapidly understand cause of failure of experiments.

Alternatively, according to a few aspects, an object of the present invention is to provide a cell observation information processing system, a cell observation information processing method, a cell observation information processing program, an archive section provided for the cell observation information processing system, and an apparatus provided for the cell observation information processing system, each of which enables users to enhance success rate of experiments.

### MEASURES TO SOLVE THE PROBLEMS

To attain the above-mentioned objects, a cell observation information processing system according to one aspect of the present invention includes: a tag acquiring section that acquires, as search tags for searching for observation information that has been acquired via an observation information acquiring section in response to an acquisition order at one time point to carry a cell observation result such as a cell image, activity data indicating cell activity by a number of cells, a cell confluency, a morphology, a survival rate or so., and an observation title, at least one of user identifying information for identifying a user who conducts cell observation, cell identifying information for identifying cells under observation, date/time information for identifying a date/time when the observation information was acquired, information on user's work for cell maintenance, apparatus identifying information for identifying an apparatus used in acquiring the observation information, activity data variation information and an image tag for indicating the cell image; a storing section that assigns the search tags acquired by the tag acquiring section to the observation information, which has been acquired in response to an acquisition order at each time point, and that stores search-tagged observation information, to which the search tags have been assigned, in an archive section; a search criterion acquiring section that acquires, as a search criterion, information containing, among the search tags assigned to the search-tagged observation information, at least one of the user identifying information, the cell identifying information, the information on user's work for cell maintenance, the apparatus identifying information, the activity data variation information, and the image tag; and a retrieving section that searches through the archive section using the search criterion acquired by the search criterion acquiring section and retrieves data containing search-tagged observation information to which search tags that are matched with the search criterion have been assigned.

A cell observation information processing method according to another aspect of the present invention includes: a tag acquiring step for acquiring, as search tags for searching for observation information that has been acquired in response to an acquisition order at one time point to carry a cell observation result such as a cell image, activity data indicating cell activity by a number of cells, a cell confluency, a morphology, a survival rate or so. , and an observation title, at least one of user identifying information for identifying a user who conducts cell observation, cell identifying information for identifying cells under observation, date/time information for identifying a date/time when the observation information was acquired, information on user's work for cell maintenance, apparatus identifying information for identifying an apparatus used in acquiring the observation information, activity data variation information and an image tag for indicating the cell image; a storing step for assigning the search tags acquired at the tag acquiring step to the observation information, which has been acquired in response to an acquisition order at each time point, and for storing search-tagged observation information, to which the search tags have been assigned, in an archive section; a search criterion acquiring step for acquiring, as a search criterion, information containing, among the search tags assigned to the search-tagged observation information, at least one of the user identifying information, the cell identifying information, the information on user's work for cell maintenance, the apparatus identifying information, the activity data variation information, and the image tag; and a retrieving step for searching through the archive section using the search criterion acquired at the search criterion acquiring step and retrieving data containing search-tagged observation information to which search tags that are matched with the search criterion have been assigned.

A cell observation information processing program according to still another aspect of the present invention makes a computer function as: a tag acquiring means that acquires, as search tags for searching for observation information that has been acquired in response to an acquisition order at one time point to carry a cell observation result such as a cell image, activity data indicating cell activity by a number of cells, a cell confluency, a morphology, a survival rate or so., and an observation title, at least one of user identifying information for identifying a user who conducts cell observation, cell identifying information for identifying cells under observation, date/time information for identifying a date/time when the observation information was acquired, information on user' s work for cell maintenance, apparatus identifying information for identifying an apparatus used in acquiring the observation information, activity data variation information and an image tag for indicating the cell image; a storing means that assigns the search tags acquired by the tag acquiring means to the observation information, which has been acquired in response to an acquisition order at each time point, and that stores search-tagged observation information, to which the search tags have been assigned, in an archive section; a search criterion acquiring means that acquires, as a search criterion, information containing, among the search tags assigned to the search-tagged observation information, at least one of the user identifying information, the cell identifying information, the information on user's work for cell maintenance, the apparatus identifying information, the activity data variation information, and the image tag; and a retrieving means that searches through the archive section using the search criterion acquired by the search criterion acquiringmeans and retrieves data containing search-tagged observation information to which search tags that are matched with the search criterion have been assigned.

### BRIEF DESCRIPTION OF THE DRAWINGS

FIG. 1 is an explanatory diagram that shows the configuration of the cell observation information processing system according to a first embodiment mode of the present invention.
FIGs. 2 are explanatory diagrams that show one layout example of tag designating screens via which a user designates search tags to be acquired by the tag acquiring section in the cell observation information processing system of FIG. 1; FIG. 2A is one layout example of the input screen for user ID, and FIG. 2B is one layout example of the input screen for cell level information, cell name, and passage number.
FIG. 3 is an explanatory diagram that shows one layout example of the search criterion designating screen via which a user designates search tags to be acquired by the search criterion acquiring section in the cell observation information processing system of FIG. 1.
FIG. 4 is an explanatory diagram that shows one example of data structure of search-tagged observation information stored by the storing section into the archive section in the cell observation information processing system of FIG. 1.
FIG. 5 is an explanatory diagram that shows one example of the relationship between observation information acquired by the observation information acquiring section and acquisition time points in the cell observation information processing system of FIG. 1.
FIG. 6 is a block diagram that shows one example of the procedure at the stage of storing observation information on cells using the cell observation information processing system of FIG. 1.
FIG. 7. is a flow chart that shows one example of the procedure at the stage of storing observation information on cells using the cell observation information processing system of FIG. 1.
FIG. 8 is a block diagram that shows one example of the procedure at the stage of searching and retrieving desired observation information on cells from the archive section.
FIG. 9 is a flow chart that shows one example of the procedure at the stage of searching and retrieving desired observation information on cells from the archive section.
FIGs. 10A-10G are graphs that individually show examples of display modes in which data containing observation information retrieved by use of the cell observation information processing system of FIG. 1 are displayed on a display surface.
FIG. 11 is an explanatory diagram that shows the configuration of the cell observation information processing system according to one modified example of FIG. 1.
FIG. 12 is an explanatory diagram that shows the configuration of the cell observation information processing system according to another modified example of FIG. 1.

### MODE FOR CARRYING OUT THE INVENTION

The embodiment mode of the present invention will be explained below. The embodiment mode explained below does not improperly limit the contents of the present invention recited in the claimed scope for a patent. In addition, not all of the configurations explained in reference to the embodiment mode below are indispensable configurations for the present invention.

A cell observation information processing system of the embodiment mode of the present invention includes: a tag acquiring section that acquires, as search tags for searching for observation information that has been acquired via an observation information acquiring section in response to an acquisition order at one time point to carry a cell observation result such as a cell image, activity data indicating cell activity by a number of cells, a cell confluency, a morphology, a survival rate or so. , and an observation title, at least one of user identifying information for identifying a user who conducts cell observation, cell identifying information for identifying cells under observation, date/time information for identifying a date/time when the observation information was acquired, information on user's work for cell maintenance, apparatus identifying information for identifying an apparatus used in acquiring the observation information, activity data variation information and an image tag for indicating the cell image; a storing section that assigns the search tags acquired by the tag acquiring section to the observation information, which has been acquired in response to an acquisition order at each time point, and that stores search-tagged observation information, to which the search tags have been assigned, in an archive section; a search criterion acquiring section that acquires, as a search criterion, information containing, among the search tags assigned to the search-tagged observation information, at least one of the user identifying information, the cell identifying information, the information on user's work for cell maintenance, the apparatus identifying information, the activity data variation information, and the image tag; and a retrieving section that searches through the archive section using the search criterion acquired by the search criterion acquiring section and retrieves data containing search-tagged observation information to which search tags that are matched with the search criterion have been assigned.

### (1) Function and effect of the configuration in which search is made by use of a tag of user identifying information (user oneself or acting worker)

As stated above, the cell observation information processing system of the embodiment mode of the present invention is configured so that the user identifying information is acquirable as a search criterion. The user identifying information is identifying information for identifying the user who conducts cell observation. To be specific, this is identifying information for identifying the very person concerned who inputs cell observation result or an acting person who conducts cell observation or inputs cell observation result in place of the very person concerned.

The cell observation information processing system of the embodiment mode of the present invention is preferably configured so that the archive section accommodates individual search-tagged observation information corresponding to cell observation by a plurality of users, and so that the search criterion acquiring section acquires information containing user identifying information as a search criterion.

If the configuration is made so that the user identifying information is acquirable as a search criterion, as in the cell observation information processing system according to the embodiment mode of the present invention, a user can retrieve observation data on cells under his or her management alone, while excluding observation data under management by others, a reference to which is not necessary, from the search result. As a result, the retrieval efficiency is improved and the time taken for cell condition checking can be shortened.

In particular, in the situation where condition checking of cells is conducted while the cells are taken outside the incubator, the shortened time for checking cell conditions can moderate damage given to the cells, and thus the effect attained by the present invention is much raised.

The configuration in which the user identifying information is acquired as a search criterion is useful in a situation where a user, upon logging in the installation, checks, in bulk, observation data on cells under his or her management alone that were acquired at a plurality of time points, for example in the case where a user reviews past observation data on cells under his or her management, before he or she conducts the work of cell condition checking of the day.

### (2) Function and effect of the configuration in which search is made by use of tags of cell identifying information (cell name, cell kind, cell level information (either "parent cell line" or "sells for experiment"), passage number)

As described above, the cell observation information processing system according to the embodiment mode of the present invention is configured so that cell identifying information is acquirable as a search criterion.

The cell identifying information is identifying information for identifying cells that are an observation target. To be specific, it is identifying information that contains at least one of: a name of the cells, a kind of the cells, cell level information for identifying whether the cells are of the parent cell line to be cultured or cells for experiment which have been separated from and cut out of the parent cell line, and a passage number of the cells.

The cell name is a name freely given by a user to the cells used for experiment and research, and the cell kind is a specific category into which the cells are classified.

The cell observation information processing system according to the embodiment mode of the present invention is preferably configured so that the archive section accommodates each set of the search-tagged observation information to which search tags adapted for cell observation directed to a plurality of cells and so that the search criterion acquiring section acquires,assearch a criterion,information containing the cell identifying information.

### (2-1) Function and effect of the configuration for search by cell name

A cell name is a name freely given by each user to cells of a parent cell line and cells for experiment subcultured therefrom to be used for experiment and research.

If the configuration is made so that a cell name is acquirable as a search criterion, as in the cell observation information processing system according to the embodiment mode of the present invention, observation data on cells of a parent cell line and cells for experiment subcultured therefrom are retrievable in bulk. As a result, a user can check a long-term variation of the conditions of the cells under culture.

In the case of handling cells that are cultured without passage, for example, the configuration for acquiring a cell name as a search criterion is useful for checking long-term variation of the conditions of the cells under culture.

### (2-2) Function and effect of the configuration for search by cell name and cell level information (either of "parent cell line" or "cells for experiment")

If the configuration is made so that a cell name and cell level information ("parent cell line" or "cells for experiment") are acquirable as a search criterion, as in the cell observation information processing system according to the embodiment mode of the present invention, observation data on cells limited to those with a certain cell name and a desired cell level ("parent cell line" or "cells for experiment") are retrievable in bulk. As a result, a user can improve efficiency of retrieval while checking a long-term variation of the cells, the cell level of which is either one of parent cell line andcells for experiment.

The configuration for acquiring a cell name and cell level information (either "parent cell line" or "cells for experiment") as a search criterion is particularly useful for sorting cells into cut-out cells (cells for experiment) and a culture from which the cells are cut out (parent cell line) and checking a long-term variation of conditions of the cells having a cell level that is either parent cell line or cells for experiment.

In general, long-term variations of cell conditions would proceed slowly. Therefore, for comparison of observation data, observation data on a parent cell line, which have been recorded continually and extensively since commencement of culture, are more convenient than observation data on cells for experiment, which were recorded as a little amount just before a formal experiment.

Therefore, according to the cell observation information processing system of the embodiment mode of the present invention, acquisition of cell level information ("parent cell line" or "cells for experiment") as a search criterion facilitates efficient retrieval of observation data that are particularly useful for recognizing cell conditions, and accordingly can shorten the time taken for checking the cell conditions by the user.

### (2-3) Function and effect of the configuration for search by cell name, cell level information (either "parent cell line" or "cells for experiment"), and passage number

If the configuration is made so that a cell name, cell level information (either "parent cell line" or "cells for experiment"), and a passage number (in addition to direct reference to a particular generation such as "3rd generation", a range of generations such as "2nd and later generations" and "5th and former generations" is available) are acquirable as a search criterion, as in the cell observation information processing system according to the embodiment mode of the present invention, a user can improve efficiency of retrieval while checking a long-term variation of cells, by limiting observation data on subcultured cells to those on cells with a certain cell name and a certain cell level ("parent cell line" or "cells for experiment") of certain generations.

The configuration for acquiring a cell name, cell level information ("parent cell line" or "cells for experiment") and a passage number as a search criterion is particularly useful for checking a long-term variation over generations (passage number) of conditions of cells that are subject to work for subculture.

### (2-4) Function and effect of the configuration for search by user identifying information and cell name

Users would individually manage naming of cells. Therefore, cell names given by a plurality of users may happen to coincide with each other.

Under such a cell management situation where a plurality of users use an identical cell name, if user identifying information is not acquired as a search criterion, observation data having an identical cell name but of a different user would be mixed in the retrieved result.

Therefore, if the configuration is made so that user identifying information and a cell name are acquirable as a search criterion, as in the cell observation information processing system according to the embodiment mode of the present invention, a user can retrieve only the observation data as intended even if an identical cell name is given by a plurality of users.

The configuration for acquiring user identifying information and a cell name as a search criterion is useful, for example in a case of handling cells that are cultured without passage, for checking a long-term variation of conditions of the cells in a situation where a plurality of users manage naming of observation data.

In addition, in the cell observation information processing system of the embodiment mode of the present invention, it is also possible to conduct a search upon using "user identifying information, cell name, and cell level information (parent cell line or cells for experiment) ", "user identifying information, cell name, cell level information (parent cell line or cells for experiment), and passage number", "user identifying information, cell name, cell level information (parent cell line or cells for experiment), and variation of activity data", or "user identifying information, cell name, and passage number" as a criterion.

### (3) Function and effect of the configuration in which search is made by use of tags of information on user's work for cell maintenance (work details, dilution ratio, container's type or size, container address where cells are contained, etc.)

As described above, the cell observation information processing system according to the embodiment mode of the present invention is configured so that information on user's work for cell maintenance is acquirable as a search criterion.

To be specific, the information on work for cell maintenance are identifying information containing at least one of work detail, dilution ratio, container's type or size, container address where the cells are contained.

### (3-1) Function and effect of the configuration for search by work detail

As stated above, a user would check conditions of cells basically every few days. By checking the cells every few days, the user can recognize a macro variation of cell conditions. Regarding cell conditions in the culture container, when it is confirmed that the cell activity has reached a predetermined level in terms of cell confluency, number of cells, etc., the user would conduct maintenance work for passage (subculture). On the other hand, the user wishes to check daily conditions of the cells during a period in which the cell activity has not yet reached the level as requires passage, by using the cell activity data.

In order to facilitate the maintenance work for passage, it is desirable that checking of the observation data every few days and checking of the observation data on a daily basis can be easily switched to each other.

Therefore, if the configuration is made so that detail of user's checking work, which is categorized into information on user's work for cell maintenance, is acquirable as a search criterion, as in the cell observation information processing system according to the embodiment mode of the present invention, a user can retrieve observation data by the detail of user's checking work. As a result, when the retrieved observation data are made displayed on display means such as a display unit, only observation data related to the checking work desired by the user are displayed while observation data on other checking work, which are unnecessary, are excluded, to shorten the time required for checking work by the user.

### (3-2) Function and effect of the configuration for search by dilution rate

Dilution rate is defined as an amount of thinning in a situation where cells cultured in a container are thinned for passage or experiment.

In the situation where cells are proliferated at a constant proliferation rate for time passage, unevenness of the dilution rate among the dates when cells are thinned is fallen within a certain range.

However, if the dilution rate greatly varies, it is probable that some status abnormality has happened to the cells due to misoperation by an acting worker, a change of proliferative behavior of cells, etc. In addition, in the case where dilution rate is large and cell confluency is low, it is, in general, not probable that the cells will grow vigorously.

Therefore, if the configuration is made so that the dilution rate, which is categorized into the information on user's work for cell maintenance, is acquirable as a search criterion as in the cell observation information processing system according to the embodiment mode of the present invention, a change in dilution rate comes to be easily detectable.

Further, in the cell observation information processing system according to the embodiment mode of the present invention, for detection of a change in dilution rate, it is more desirable to configure a search so that another search criterion such as a deviation of the dilution rate from an average value is usable.

### (3-3) Function and effect of the configuration for search by container's type or size

In culturing cells, users often use different types or sizes of containers in accordance with uses. For example, in a case where subculture is to be carried out, some user would use a flask for a parent cell line and dishes or wells for cells for experiment.

Therefore, in such a case, for example, if the configuration is made so that container's type or size is acquirable as a search criterion as in the cell observation information processing system according to the embodiment mode of the present invention, observation data on the parent cell line alone are retrievable by carrying out a search by "flask", whereas observation data on cells for experiment alone are retrievable by carrying out a search by "dish" or "well".

In cases where cells are cultured for the purpose for multiplying number of the cells, it is a customary practice to replace the container to larger ones one after another as the culturing proceeds. For example, in the early stage of cell culture, the cells are contained in a small culture container, and, as the culturing gradually proceeds, the cells are transplanted and cultured in a larger culture container.

Therefore, in such cases, if the configuration is made so that container's type or size is acquirable as a search criterion as in the cell observation information processing system according to the embodiment mode of the present invention, observation data on the early stage of cell culture are retrievable by acquiring "small size", whereas observation data on cells that have proceeded to a certain stage under culture are retrievable by acquiring "large size".

### (3-4) Function and effect of the configuration for search by container address where the container is stored

Further, in the cell observation information processing system according to the embodiment mode of the present invention, the configuration can be made so that a container address (such as a well address) where the container is stored is acquirable as a search criterion.

In general, wells are used as containers for culturing cells for experiment that are to be used for experiments.

In a case where cells for experiment are to be cultured, a small amount of the cells are planted in a well and cultured for few days until the cells are stably attached to the well. There is a demand for observation of changes during this period.

Thus, if the configuration is made so that a well address is acquirable as a search criterion as in the cell observation information processing system according to the embodiment mode of the present invention, a result of a formal experiment regarding cells at a certain address in the wells can be considered as being associated with observation data on this address, so that accuracy of analysis of the result is improved.

In the configuration where it is possible to acquire, as a search criterion, details of user's checking work, which is categorized into information on user's work for cell maintenance in the cell observation information processing system according to the embodiment mode of the present invention, it is desirable, in general, to conduct a search using not mere "details of user's checking work" but "user identifying information and details of user's checking work" as a search criterion.

### (4) Function and effect of the configuration in which search is made by use of a tag of apparatus identifying information for identifying apparatus

As stated above, the cell observation information processing system according to the embodiment mode of the present invention is configured so that apparatus identifying information is acquirable as a search criterion.

If the configuration is made so that apparatus identifying information is acquirable as a search criterion as in the cell observation information processing system according to the embodiment mode of the present invention, in a case of a system configuration in which cells are observed and the observation data are recorded by use of a plurality of apparatuses, a search on the basis of apparatus makes it possible to acquire image capture situations specific to individual apparatuses, and accordingly the cause of experiment failure depending on image capture situations specific to individual apparatuses can be recognized promptly and smoothly.

### (5) Function and effect of the configuration in which search is made by use of activity data variation information

As stated above, the cell observation information processing system according to the embodiment mode of the present invention is configured so that activity data variation information is acquirable as a search criterion.

The activity data variation information is expressed, for example by an amount of variation (thatis, a rate of variation) between respective activity data (for example, cell confluency, number of cells or survival rate) at a plurality of time points, and can be shown, for example by a slope of a plotted line in a graph.

For example in a graph that shows variation information on number of cells, it is probable that cells in a certain period of observation information acquisition that corresponds to a part of the graph where the variation rate of the number of cells is large (the slope of the plotted line is steep) are more vigorously proliferated than cells during a period of observation information acquisition that corresponds to a part where the variation rate of the number of cells is small (the slope of the plotted line is moderate).

Therefore, if the configuration is made so that activity data variation information is acquirable as a search criterion as in the cell observation information processing system according to the embodiment mode of the present invention, observation data on cells that are vigorously proliferated are solely retrievable. As a result, upon selecting cells of an observation information acquisition period that is found out from the observation data as a period during which cells are vigorously proliferated, users can use the cells of this observation information acquisition period alone for a formal experiment, to raise success rate of the formal experiment.

### (6) Function and effect of the configuration in which search is made by use of an image tag

As stated above, the cell observation information processing system according to the embodiment mode of the present invention is configured so that an image tag is acquirable as a search criterion.

The image tag is a tag for indicating a cell image, to be specific, an image displayable in a small size in an input screen or information containing an image and its corresponding name integrally combined together.

If the configuration is made so that an image tag is acquirable as a search criterion as in the cell observation information processing system according to the embodiment mode of the present invention, for example in a case where the data structure is built so that user identifying information, cell identifying information, date/time information, information on user' s work for cell maintenance, apparatus identifying information, activity data variation information etc. are subordinate to an image tag in search-tagged observation information, the user can acquire user identifying information, cell identifying information, date/time information, information on user's work for cell maintenance, apparatus identifying information, activity data variation information etc. in bulk as search tags only by selecting and inputting an image tag alone without designating and inputting individual search tags in the selectable input screen, and accordingly can search desired observation data easily and promptly.

In addition, the cell observation information processing system according to the embodiment mode of the present invention is preferably configured as follows.

The observation information is preferably constructed of information that carries a plurality of observation results corresponding to image capture at one time point directed to a plurality of observation positions on a specimen containing the cells.

The information carrying the plurality of observation results preferably contains a plurality of cell images.

Alternatively, the information carrying the plurality of observation results preferably contains a value calculated on the basis of a plurality of cell images.

Alternatively, the information carrying the plurality of observation results preferably contains a plurality of cell images and a value calculated on the basis of the plurality of cell images.

Alternatively, the information carrying the plurality of observation results preferably contains a plurality of cell images, and the search tags include a value calculated on the basis of the plurality of cell images.

Alternatively, the information carrying the plurality of observation results preferably contains a plurality of cell images, and the search tags include an image tag for indicating the plurality of cell images.

Alternatively, the information carrying the plurality of observation results preferably contains a plurality of cell images, and the search tags include a value calculated on the basis of the plurality of cell images.

Alternatively, the information carrying the plurality of observation results preferably contains a plurality of cell images, and the search tags include an image tag for indicating the plurality of cell images and a value calculated on the basis of the plurality of cell images.

Such a configuration allows the user to more accurately grasp the cell conditions even if the cells are unevenly distributed in a container.

As the value calculated on the basis of the plurality of cell images, for example an average of cell activity data in terms of cell confluency, number of cells, survival rate or so is applicable.

Hereinafter, an explanation is made on the embodiment mode of the present invention in reference to the drawings.

### First embodiment mode

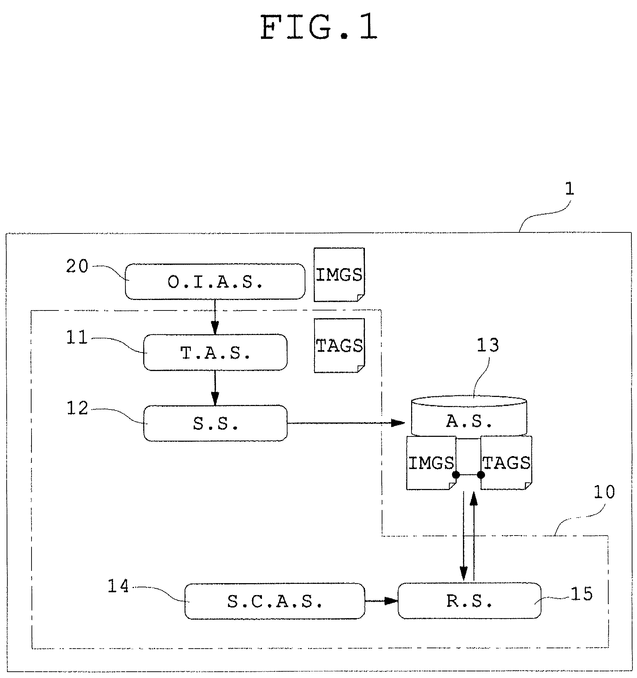

FIG. 1 is an explanatory diagram that shows the basic configuration of the cell observation information processing system according to a first embodiment mode of the present invention.

The cell observation information processing system 10 of the first embodiment mode includes, as shown in FIG. 1, a tag acquiring section 11, a storing section 12, a search criterion acquiring section 14 and a retrieving section 15. In FIG. 1, the reference numeral 1 denotes a cell observation information processing installation, the reference numeral 13 denotes an archive section, and the reference numeral 20 denotes an observation information acquiring section. In FIG. 1, the cell observation information processing system 10 is configured as a stand-alone system with the tag acquiring section 11, the storing section 12, the search criterion acquiring section 14 and the retrieving section 15 being provided in the single installation 1 together with the archive section 13 and the observation information acquiring section 20. The cell observation information processing installation 1 is configured, for example of a microscope apparatus provided with an image capture apparatus and a computer provided with a database.

The tag acquiring section 11 is configured to acquire, as search tags for searching for observation information each acquired via the observation information acquiring section 20 in response to an acquisition order at one time point, user identifying information, cell identifying information, date/time information for identifying the date/time when the observation information was acquired, information on user' s work for cell maintenance, apparatus identifying information, activity data variation information and an image tag.

The observation information, the user identifying information, the cell identifying information, the information on user's work for cell maintenance, the apparatus identifying information, and the image tag are explained above.

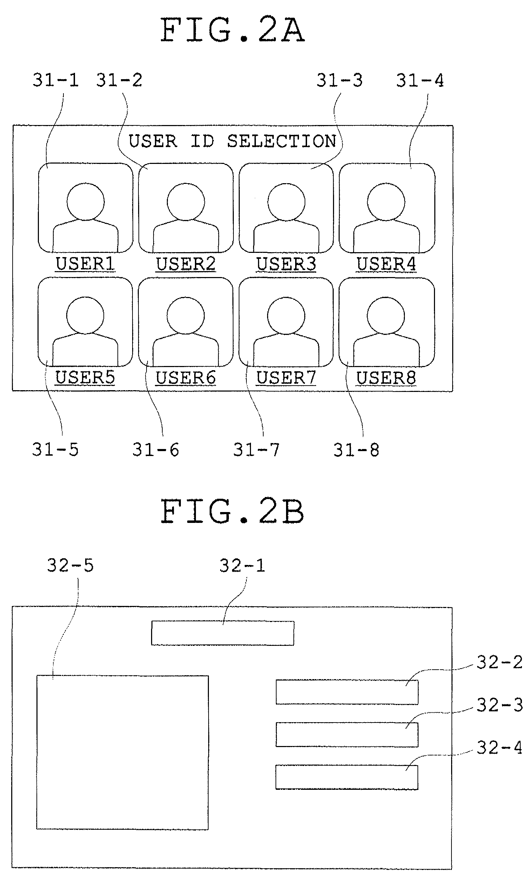

To be specific, the tag acquiring section 11 is configured to acquire, as search tags, for example a user ID as user identifying information selected and inputted by a user in a user ID input screen shown in FIG. 2A provided in the cell observation information processing installation 1 and for example cell level information ("parent cell line" or "cells for experiment"), a cell name and a passage number inputted by the user in a search tag input screen shown in FIG. 2B provided in the cell observation information processing installation 1. The tag acquiring section 11 may be configured to automatically acquire preset values of the cell level information ("parent cell line" or "cells for experiment"), the cell name and the passage number without via input by the user in the tag input screen shown in FIG. 2B.

Also, the tag acquiring section 11 acquires image capture date/time having been recorded as a log in cell images in each set of observation information acquired via the observation information acquiring section 20, as date/time information for identifying the date/time when the observation information was acquired. Also, the tag acquiring section 11 acquires an image tag from cell images in each observation information acquired via the observation information acquiring section 20.

While the user ID is used as the user identifying information in this embodiment mode for convenience' sake, the user identifying information is not necessarily limited to the user ID; any letters or symbols that can distinguish a certain user from other users, such as a name or nickname of the user, may work.

In FIG. 2A, the reference numerals 31-1 to 31-n (in FIG. 2A, n = 8 for convenience' sake) denote user-ID selectable input sections. Each of the user-ID selectable input sections 31-1 to 31-n is configured of an image tag displayed to be selectable for input and has a form of an image of a user and text information of the user ID integrally combined together. In FIG. 2B, the reference numeral 32-1 denotes a user-ID display field, the reference numeral 32-2 denotes a cell level information input field, the reference numeral 32-3 denotes a cell name input field, the reference numeral 32-4 denotes a passage number input field, and the reference numeral 32-5 denotes an observation data display field. Each of the input fields 32-2 to 32-4 is configured so that a user can input desired information therein. It is noted that each of the input fields 32-2 to 32-4 may be configured so that a user can select and input information among several possible values. The user ID display field 32-1 is configured to display the user ID selected and inputted in the user ID input screen. The observation data display field 32-5 is configured to display the observation information (for example, activity data) of cells acquired by the observation information acquiring section 20.

The storing section 12 is configured to assign the search tags acquired by the tag acquiring section 11 to each set of observation information acquired by the observation information acquiring section 20 in response to an acquisition order at each time point, and to store the search-tagged observation information, to which the search tags are assigned, in the archive section 13.

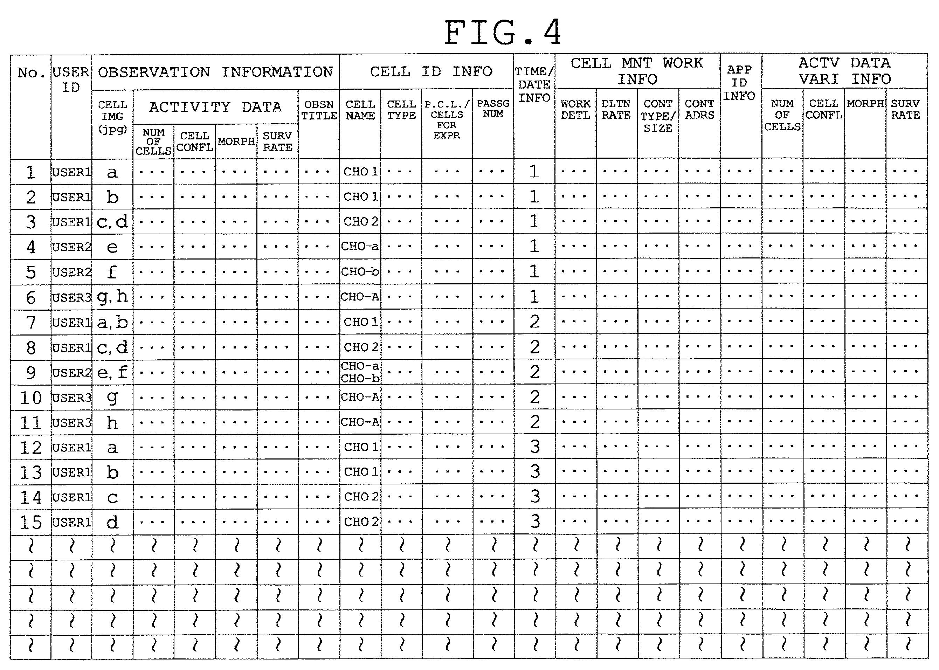

The archive section 13 has, for example as shown in FIG. 4, one or more records of the search-tagged observation information. Each record is created corresponding to image capture at one time point of cell images, where observation information corresponding to the image capture at one time of the cell images is interrelated with search tags for retrieving the observation information.

The search criterion acquiring section 14 is configured to acquire, as a search criterion, information containing at least one of the tags of user identifying information, cell identifying information, information on user's work for cell maintenance, apparatus identifying information, activity data variation information and an image tag, out of the search tags assigned to the search-tagged observation information.

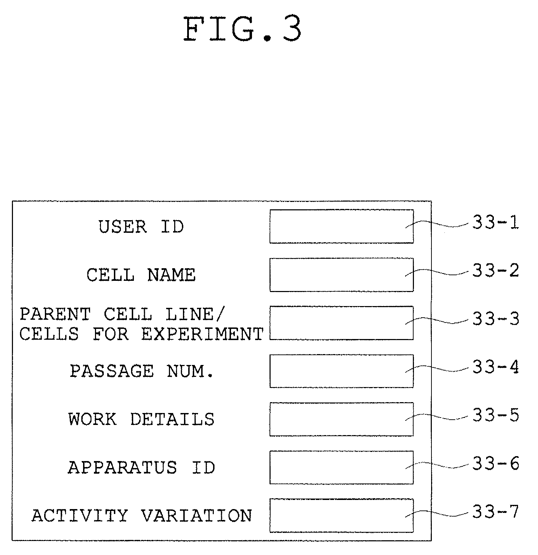

To be specific, the search criterion acquiring section 14 is configured to acquire, as a search criterion, for example a combination of activity data and date/time information as added to the information on the items for which the user has made some input, out of the items of user ID as user identifying information, cell name as cell identifying information, cell level information ("parent cell line" or "cells for experiment"), passage number, work detail as information on user' s work for cell maintenance, apparatus ID as apparatus identifying information, and activity variation amount as activity data variation information presented for example in the search criterion designating screen as shown in FIG. 3 provided in the installation 1. It is noted that the search criterion acquiring section 14 may be configured to acquire only the information inputted by the user in the search criterion designating screen. Also, the search criterion acquiring section 14 may be configured to automatically acquire preset values of information such as user ID, cell name, cell level information ("parent cell line" or "cells for experiment"), passage number, work details, apparatus ID and activity data variation amount, without via input by the user in the search criterion designating screen shown in FIG. 3.

In FIG. 3, the reference numeral 33-1 denotes a user ID input field, the reference numeral 33-2 denotes a cell name input field, the reference numeral 33-3 denotes a cell level information ("parent cell line" or "cells for experiment") input field, the reference numeral 33-4 denotes a passage number input field, the reference numeral 33-5 denotes a work detail input field, the reference numeral 33-6 denotes an apparatus ID input field, and the reference numeral 33-7 denotes an activity variation amount input field. Each of the input fields 33-1 to 33-7 is configured so that a user can input desired information therein. It is noted that each of the input fields 33-1 to 33-7 may be configured so that a user can select and input information among several possible values.

Although the search criterion designating screen of the FIG.3 example does not provide an input field to input therein a cell kind as the cell identifying information, it may be configured so that an input field for inputting a cell kind therein is provided and so that the search criterion acquiring section 14 acquires the inputted cell kind as a search criterion, as a matter of course. Further, the search criterion designating screen of FIG. 3 is shown as a mere example; it may be configured to set, as input items for search tags searchable by the search criterion acquiring section 14, any combination of desired items in subcategories in the user identifying information, the cell identifying information, the date/time information for identifying date/time when the observation information was acquired, the information on user's work for cell maintenance, the apparatus identifying information, the activity data variation information and the image tag (for example, cell name, cell kind, "cells for experiment" or "parent cell line", passage number etc. fall in subcategories in the cell identifying information), as a matter of course.

Also, the user ID input field 33-1 in the search criterion designating screen of the FIG. 3 example may be configured to initially display the user ID that has been selected and inputted by the user in the user ID input screen. In this case, a user ID display field may be provided in place of the user ID input field 33-1, just to display the user ID having been selected and inputted by the user in the user ID input screen, so that input of a user ID by the user is omitted.

The retrieving section 15 is configured to search through the archive section 13 using the search criterion acquired by the search criterion acquiring section 14 and to retrieve, from the archive section 13, data containing the search-tagged observation information to which search tags matched with the search criterion are assigned.

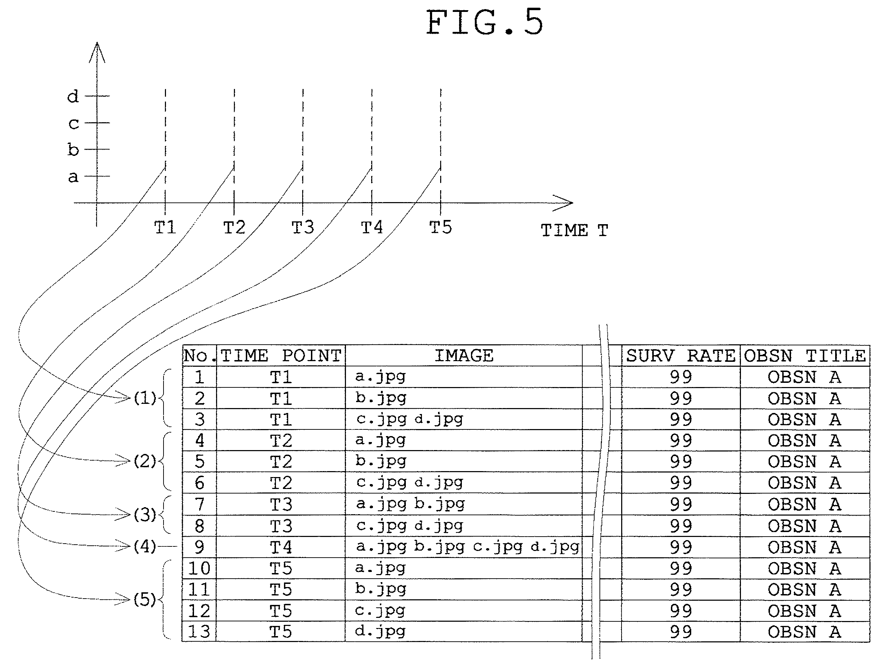

The observation information acquiring section 20 is configured to acquire observation information in response to an acquisition order at one time point. To be specific, the observation information acquiring section 20 is actuated when a user inputs an observation name and pushes an order button for observation information acquisition on an image-capture order screen (not shown in the drawings), makes an image capture apparatus (not shown in the drawings) provided in the installation 1 image-capture a specimen containing cells placed at an observation position, detects activity data that show cell activity in terms of number of cells, cell confluency, morphology, survival rate or so by subjecting a cell image as captured to image processing and analysis processing via an image processing section and an image analyzing section (not shown in the drawings), and, as shown in FIG. 5, temporally stores, in a storage area (not shown in the drawings) in the installation 1, the cell image, the activity data and the observation name as one set of observation information. In the case of FIG. 5 example, a group (1) of records numbered 1 to 3 form one set of observation information acquired by the observation information acquiring section 20 in response to an acquisition order at a time point T1. Similarly, a group (2) of records numbered 4 to 6, a group (3) of records numbered 7 and 8, a group (4) of record numbered 9, and a group (5) of records numbered 10 to 13 form individual sets of observation information acquired by the observation information acquiring section 20 in response to acquisition orders at different time points T2 to T5, respectively.

The cell observation information processing installation 1 is provided with a system log-in screen (not shown in the drawings). If a user inputs a log-in ID and a password of the system in the system log-in screen, a user-ID input screen as shown in FIG. 2A is displayed.

In the user-ID input screen, if the user inputs his or her user ID as the user identifying information by selection among the user-ID selectable input sections 31-1 to 31-n, the user ID is temporally stored in the storage area (not shown in the drawings) in the installation 1 and an image capture order screen (not shown in the drawings) is displayed so that the user can perform operation for image-capture order. The user ID input screen of FIG. 2A may be configured to function as a system log-in screen also by letting a user perform input operation for a log-in ID and a password of the system together with select and input operation for a user ID.

Further, the cell observation information processing system 10 is preferably provided with a display surface (not shown in the drawings) as a display section for displaying data containing search-tagged observation information retrieved by the retrieving section 15.

An explanation is made on a processing procedure of condition management of cells using the cell observation information processing system according to the first embodiment mode thus configured.

The procedure of condition management of cells cultured by users is divided into a stage of storing observation information on the cells under culture into the archive section and a stage of searching and retrieving desired observation information on cells from the archive section and checking conditions of the cells.

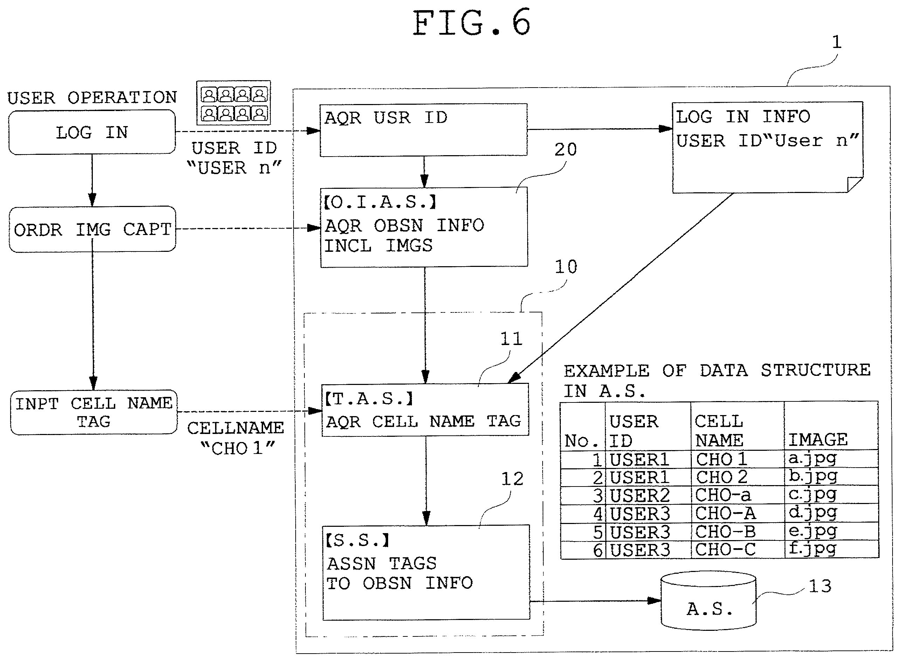

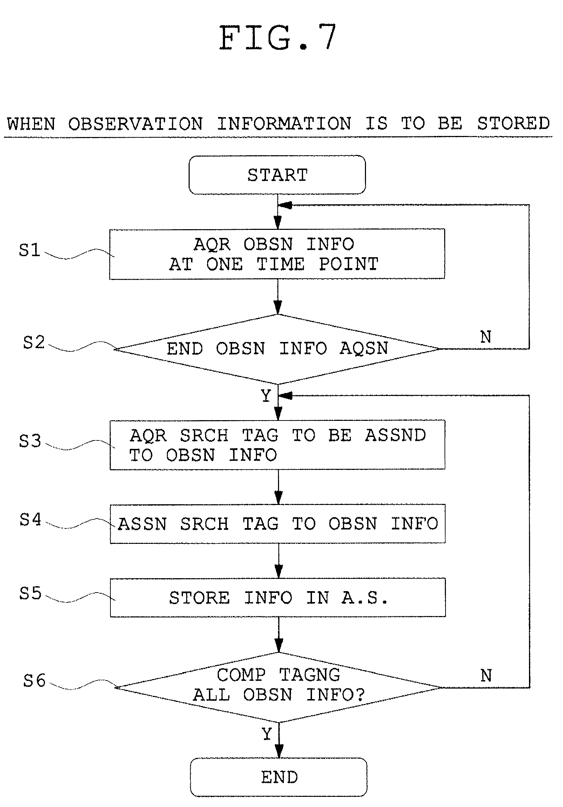

First, the processing procedure at the stage of storing observation information on cells under culture into the archive section is explained in reference to FIGs. 6 and 7. In an example of data structure in the archive section shown in FIG. 6, only a part of data items are shown for convenience' sake.

The user inputs a log-in ID and a password of the system in the system log-in screen (not shown in the drawings). Then, the user ID input screen as shown in FIG. 2A is displayed.

Then, the user inputs his or her own user ID by selection among the user-ID selectable input sections 31-1 to 31-n. Consequently, the user ID is temporally stored in the storage area in the installation 1 and an image-capture order screen (not shown in the drawings) is displayed, to allow the user to perform operation for image-capture order.

Then, in the image-capture order screen (not shown in the drawings), the user inputs an observation name and pushes an order button for ordering acquisition of observation information. Then, the observation information acquiring section 20 makes the image capture apparatus (not shown in the drawings) image-capture a specimen containing cells placed at the observation position, detects activity data that show cell activity in terms of number of cells, cell confluency, morphology, survival rate or so, for example by subjecting a cell image as captured to image processing and analysis processing via the image processing section and the image analyzing section (not shown in the drawings), and temporally stores, in the storage area (not shown in the drawings) in the installation 1, the cell image, the activity data and the observation name as one set of observation information. In this way, the observation information at one time point is acquired (Step S1). It is noted that acquisition of observation information can be repeated a plurality of times for different acquisition time points (Step S2).

Upon completion of input and observation information acquisition order by the user from the image-capture order screen and acquisition of observation information by the observation information acquiring section 20, the user inputs "parent cell line" or "cells for experiment", a cell name and a passage number in the search tag input screen as shown in FIG. 2B. When the input completes, the tag acquiring section 11 acquires, as search tags, "parent cell line" or "cells for experiment", the cell name and the passage number, which have been inputted by the user in the tag input screen, and the user ID, which has been inputted by the user in the user ID selective input screen (Step 3). In the example of FIG. 7, the configuration is made so that, after a plurality of times of repetition of observation information acquisition by the observation information acquiring section 20, the display section is switched to the mode for inputting tags in the search tag input screen and acquiring the search tags by the tag acquiring section 11. However, the configuration may be made so that the display section is switched each time after observation information is acquired by the observation information acquiring section, to the mode for inputting tags in the search tag input screen and acquiring the search tags by the tag acquiring section 11. Regarding timing of assigning the search tags, the configuration may be made so that the display section is switched to the mode for inputting tags in the search tag input screen and acquiring the search tags by the tag acquiring section 11 in real time after acquisition of observation information by the observation information acquiring section 20, or so that the display section is switched to the mode for inputting tags in the search tag input screen and acquiring the search tags by the tag acquiring section 11 a certain time after acquisition of observation information by the observation information acquiring section 20.

After that, the storing section 12 assigns the search tags acquired by the tag acquiring section 11 to individual sets of observation information acquired in response to acquisition orders at individual time points (Step S4), and stores the search-tagged observation information, to which the search tags are assigned, into the archive section 13 (Step S5).

Upon completion of assignment of tags to observation information at every time point and storage of the search-tagged observation information into the archive section 13, the procedure at the stage of storing observation information on cells under culture into the archive section 13 finishes (Step S6).

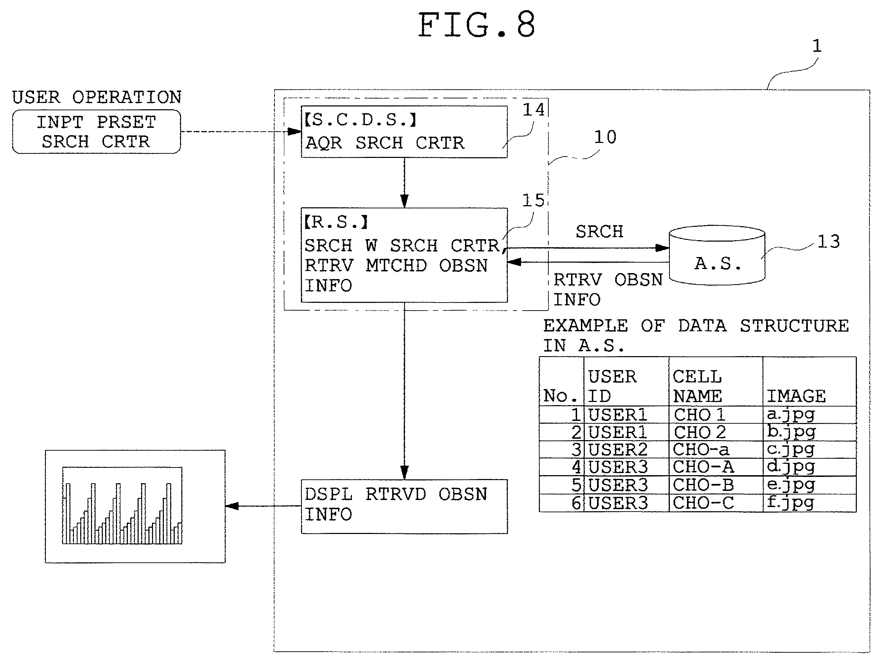

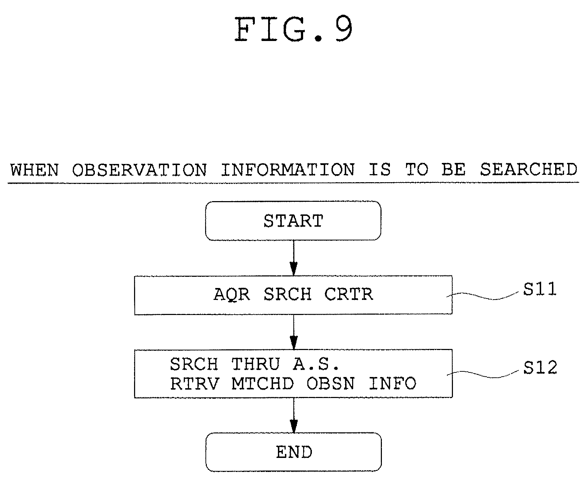

Next, the processing procedure at the stage of searching and retrieving desired observation information on cells from the archive section is explained in reference to FIG. 8 and FIG. 9. In an example of data structure in the archive section shown in FIG. 8, only a part of data items are shown for convenience' sake.

In the search criterion designating screen as shown in FIG. 3, the user designates, as a search criterion, information containing, among the search tags assigned to the search-tagged observation information, at least one of a user ID as user identifying information, a cell name as cell identifying information, cell level information ("parent cell line" or "cells for experiment"), work detail as information on user's work for cell maintenance, an apparatus ID as apparatus identifying information, and an activity rate as activity data variation information. Upon completion of the input, the search criterion acquiring section 14 acquires, as a search criterion, for example a combination of activity data and date/time information as added to the information inputted by the user in the search criterion designating screen (Step S11). As stated above, the field of user ID in the search criterion designating screen of FIG. 3 may be configured to initially display the user ID that has been selected and inputted by the user in the user ID input screen, to omit input by the user.

Next, the retrieving section 15 searches through the archive section 13 using the search criterion acquired by the search criterion acquiring section 14, and retrieves data containing search-tagged observation information to which search tags matched with the search criterion have been assigned (Step S12). Whereby, the procedure of searching and retrieving desired observation data on cells from the archive section is completed.

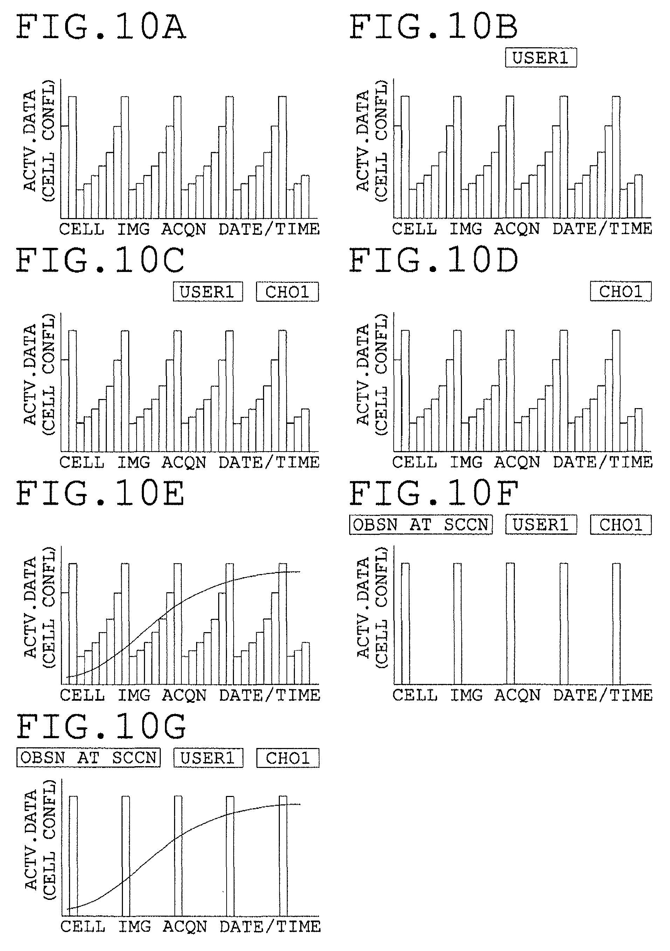

The data containing search-tagged observation information retrieved by the retrieving section 15 is displayed on a display surface (not shown in the drawings) in a display mode that depends on the search criterion acquired by the search criterion acquiring section 14. The user checks cell conditions by observing the data displayed on the display surface for example in a displaymode as shown in FIGs. 10A-10G. Whereby, the procedure at the stage of searching and retrieving desired observation information on cells from the archive section and checking the cell conditions is completed.

FIGs. 10A-10G are graphs that show, as examples of display mode on the display surface for presenting to a user data containing the retrieved observation information, out of the retrieved data containing observation information at individual time points, cell confluency as activity data (bar graphs) and/or cumulated number of times of cell division (line graphs) calculated out on the basis of number of cells measured at each time point, plotted in bulk in order of date/time at which the observation information were acquired over several generations. The display surface shown in FIGs. 10A-10G may be configured so that the screen is switchable to a desired display mode by user operation. The display screens shown in FIGs. 10A-10B are mere examples of display modes of the display screen for presenting retrieved data containing observation information before users, and thus are not limited to these display modes, as a matter of course.

According to the cell observation information processing system 10 of the first embodiment mode, since the configuration is made so that the user identifying information is acquirable as a search criterion, a user can retrieve observation data on cells under his or her management alone, while excluding observation data under management by others, a reference to which is not necessary, from the search result. As a result, the retrieval efficiency is improved and the time taken for cell condition checking can be shortened.

In particular, in the situation where condition checking of cells is conducted while the cells are taken outside the incubator, the shortened time for checking cell conditions can moderate damage given to the cells.

According to the cell observation information processing system 10 of the first embodiment mode, since the configuration is made so that cell identifying information is acquirable as a search criterion, for example in a case where the configuration is made so that a cell name is acquirable as a search criterion, observation data on cells of a parent cell line and its cells for experiment are retrievable in bulk. As a result, a long-term variation of the cells under culture can be checked.

Also, for example in a case where the configuration is made so that a cell name and cell level information ("parent cell line" or "cells for experiment") are acquirable as a search criterion, observation data on cells limited to those with a certain cell name and a desired cell level ("parent cell line" or "cells for experiment") are retrievable in bulk. As a result, a user can improve efficiency of retrieval while checking a long-term variation of the cells, the cell level of which is either one of parent cell line and cells for experiment.

Also, for example in a case where the configuration is made so that a cell name, cell level information (either "parent cell line" or "cells for experiment"), and a passage number (in addition to direct reference to a particular generation such as "3rd generation", a range of generations such as "2nd and later generations" and "5th and former generations" is available) are acquirable as a search criterion, a user can improve efficiency of retrieval while checking a long-term variation of cells, by limiting observation data on subcultured cells to those on cells with a certain cell name and a certain cell level ("parent cell line" or "cells for experiment") of certain generations.

Also, for example in a case where the configuration is made so that a user ID and a cell name are acquirable as a search criterion, a user can retrieve only the observation data as intended even if an identical cell name is given by a plurality of users.

According to the cell observation information processing system 10 of the first embodiment mode, since the configuration is made so that information on user's work for cell maintenance is acquirable as a search criterion, for example in a case where the configuration is made so that details of user's checking work are acquirable as a search criterion, a user can retrieve observation data by the detail of user' s checking work. As a result, when the retrieved observation data are made displayed on display means such as a display unit, only observation data related to the checking work desired by the user are displayed while observation data on other checking work, which are unnecessary, are excluded, to shorten the time required for checking work by the user.

Also, for example in a case where the configuration is made so that dilution rate is acquirable as a search criterion, a change in dilution rate comes to be easily detectable.

In culturing cells, users often use different types or sizes of container in accordance with uses. Therefore, for example in a case where the configuration is made so that container's type or size is acquirable as a search criterion, observation data in accordance with uses are efficiently retrievable by conducting a search by container's type or size.

Also, for example in a case where the configuration is made so that a container address is acquirable as a search criterion, a result of a formal experiment regarding cells at a certain address in the container can be considered as being associated with observation data on this address, so that accuracy of analysis of the result is improved.

According to the cell observation information processing system 10 of the first embodiment mode, since the configuration is made so that apparatus identifying information is acquirable as a search criterion, in a case of a system configuration in which cells are observed and the observation data are recorded by use of a plurality of apparatuses, a search on the basis of apparatus makes it possible to acquire image capture situations specific to individual apparatus, and accordingly the cause of experiment failure depending on image capture situations specific to individual apparatuses can be recognized promptly and smoothly.

According to the cell observation information processing system 10 of the first embodiment mode, since the configuration is made so that activity data variation information is acquirable as a search criterion, observation data on cells that are vigorously proliferated are solely retrievable. As a result, upon selecting cells of an observation information acquisition period that is found out from the observation data as a period during which cells are vigorously proliferated, users can use the cells of this observation information acquisition period alone for a formal experiment, to raise success rate of the formal experiment.

According to the cell observation information processing system 10 of the first embodiment mode, since the configuration is made so that an image tag is acquirable as a search criterion, for example in a case where the data structure is built so that user identifying information, cell identifying information, date/time information, information on user's work for cell maintenance, apparatus identifying information, activity data variation information etc. are subordinate to an image tag in search-tagged observation information, the user can acquire user identifying information, cell identifying information, date/time information, information on user's work for cell maintenance, apparatus identifying information, activity data variation information etc. in bulk as search tags only by selecting and inputting an image tag alone without designating and inputting individual search tags in the selectable input screen, and accordingly can search desired observation data easily and promptly.

While the embodiment mode of the cell information processing system according to the present invention is explained above, the cell information processing system may be configured of a cell observation information processing program that makes a computer provided in the installation 1 function as: a tag acquiring means that acquires, as search tags for searching for observation information that has been acquired in response to an acquisition order at one time point to carry a cell observation result such as a cell image, activity data indicating cell activity by a number of cells, a cell confluency, a morphology, a survival rate or so., and an observation title, at least one of user identifying information for identifying a user who conducts cell observation, cell identifying information for identifying cells under observation, date/time information for identifying a date/time when the observation information was acquired, information on user's work for cell maintenance, apparatus-identifying information for identifying an apparatus used in acquiring the observation information, activity data variation information and an image tag for indicating the cell image; a storing means that assigns the search tags acquired by the tag acquiring means to the observation information, which has been acquired in response to an acquisition order at each time point, and that stores search-tagged observation information, to which the search tags have been assigned, in an archive section; a search criterion acquiring means that acquires, as a search criterion, information containing, among the search tags assigned to the search-tagged observation information, at least one of the user identifying information, the cell identifying information, the information on user's work for cell maintenance, the apparatus identifying information, the activity data variation information, and the image tag; and a retrieving means that searches through the archive section using the search criterion acquired by the search criterion acquiring means and retrieves data containing search-tagged observation information to which search tags that are matched with the search criterion have been assigned.

Also, the cell observation information processing system according to the embodiment mode of the present invention may have, other than the configuration in which a cell observation information processing program is provided in a computer provided in the installation 1, for example a configuration as recorded in a recording medium readable by a computer provided in the installation 1.

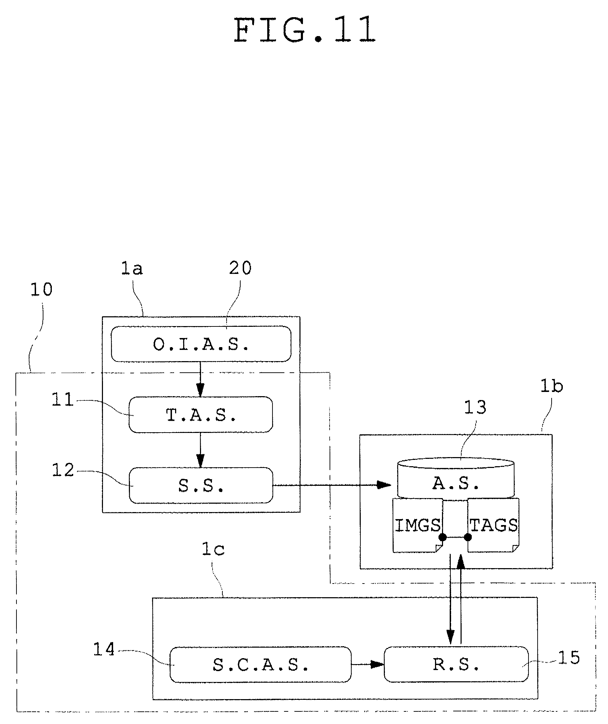

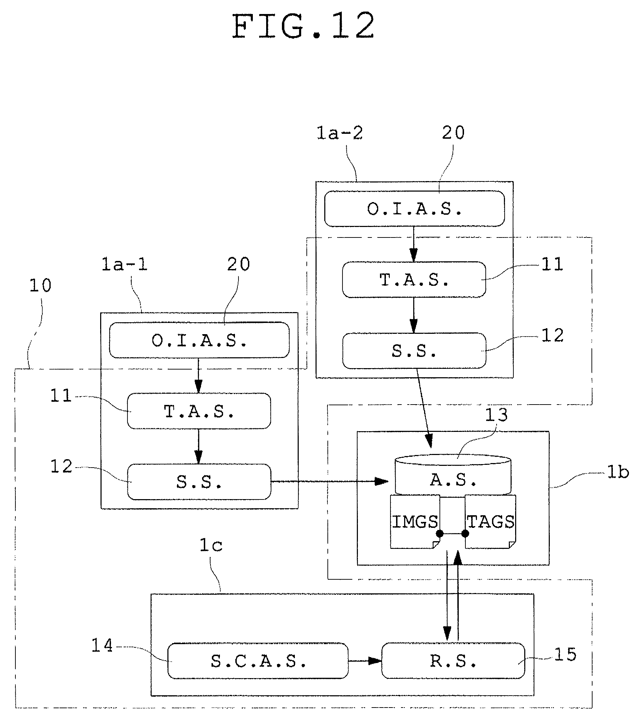

The cell observation information processing system according to the embodiment mode of the present invention is not limited to the configuration in which the tag acquiring section 11, the storing section 12, the search criterion acquiring section 14 and the retrieving section 15 are provided in one installation 1 as shown in FIG. 1, but may be configured so that, for example as shown in FIGs. 11 and 12 as modified examples, the tag acquiring section, the storing section, the search criterion acquiring section and the retrieving section are separately arranged in a plurality of apparatuses. Also, the cell observation information processing system according to another embodiment mode of the present invention may be configured to include the archive section 13 also together with the tag acquiring section 11, the storing section 12, the search criterion acquiring section 14 and the retrieving section 15.

FIG. 11 is an explanatory diagram that shows the configuration of the cell observation information processing system according to one modified example of FIG. 1.

The cell observation information processing system 10 is configured so that the tag acquiring section 11 and the storing section 12 are provided in an observation processing apparatus 1a, the archive section 13 is provided in an archive processing apparatus 1b, and the search criterion acquiring section 14 and the retrieving section 15 are provided in a search processing apparatus 1c. The observation information acquiring section 20 is provided in the observation processing apparatus 1a.

The observation processing apparatus 1a is configured, for example of a microscope apparatus provided with an image capture apparatus and a computer.

The archive processing apparatus 1b is configured, for example of a storage unit that stores database files.

The search processing apparatus 1c is configured, for example of a computer provided with a database software.

The observation processing apparatus 1a and the archive processing apparatus 1b, and the archive processing apparatus 1b and the search processing apparatus 1c are interconnected in network, respectively.

The configurations of the tag acquiring section 11, the storing section 12, the archive section 13, the search criterion acquiring section 14, the retrieving section 15 and the observation information acquiring section 20 are substantially the same as the configurations in the first embodiment mode shown in FIG. 1.

In the cell observation information processing system 10 of FIG. 11 thus configured, at the stage of storing observation information on cells under culture into the archive section 13, the observation information acquiring section 20 provided in the observation processing apparatus 1a acquires observation information in response to an acquisition order at one time point by a user. Then, the tag acquiring section 11 acquires search tags for searching the observation information acquired via the observation information acquiring section 20. Then, the storing section 12 assigns the search tags acquired by the tag acquiring section 11 to the observation information, and stores the search-tagged observation information into the archive section 13 in the archive apparatus 1b via a network line.

At the stage of searching and retrieving desired observation information on cells from the archive section 13 and checking cell conditions, the search criterion acquiring section 14 in the search processing apparatus 1c acquires a search criterion inputted on a search criterion designating screen (not shown in the drawings) by the user. Then, the retrieving section 15 searches through the archive processing apparatus 1b via the network line using the search criterion acquired by the search criterion acquiring section 14, to retrieve data containing search-tagged observation information to which search tags matched with the search criterion are assigned. The retrieved data containing search-tagged observation information are displayed on a display unit not shown in the drawings.

According to the cell observation information processing system of FIG. 11, since the tag acquiring section 11, the storing section 12, the search criterion acquiring section 14 and the retrieving section 15, which constitute the cell observation information processing system 10, are dividedly provided in a plurality of apparatuses 1a, 1b and 1c, the individual apparatuses can be made small-sized and simple.

Since the tag acquiring section 11 plus the storing section 12 and the search criterion acquiring section 14 plus the retrieving section 15 are provided in the separate apparatuses 1a and 1c, the user can use different operation sites at the stage of storing observation information on cells under culture into the archive section 13 and at the stage of searching and retrieving desired observation information on cells from the archive section 13 and checking cell conditions, to enjoy improved convenience. For example, by using a mobile apparatus for the search processing apparatus 1c, without being tied to the location of the observation information acquiring apparatus 1a, the user can easily search and retrieve data containing desired observation information from the archive section 13 via network communication, to check cell conditions.

It is noted that the tag acquiring section 11 and the storing section 12 may be provided in an apparatus different from the observation information acquiring section 20.

If the tag acquiring section 11 and the storing section 12 are provided in an apparatus different from the observation information acquiring section 20, the user can use different operation sites at the observation information acquiring stage and at the tag acquiring stage, to enjoy much improved convenience. For example, by using a mobile apparatus for the apparatus provided with the tag acquiring section 11 and the storing section 12, without being tied to the location of the observation information acquiring apparatus 1a, the user can easily assign desired search tags to the observation information acquired by the observation information acquiring section 20 and record them in the archive section 13 via network communication, by making the mobile apparatus display a search tag input screen as shown in FIG. 2B and inputting the desired search tags.

It is noted that the search criterion acquiring section 14 and the retrieving section 15 may be provided integrally in the observation processing apparatus 1a.

FIG. 12 is an explanatory diagram that shows the configuration of the cell observation information processing system according to another modified example of FIG. 1.

The cell observation information processing system 10 of this modified example is configured so that a plurality of observation processing apparatuses 1a-1 to 1a-n (in FIG. 12, n = 2 for convenience' sake) acquire observation information, and the observation information acquired by the respective observation processing apparatuses 1a-1 to 1a-n are managed in bulk.

To be specific, in the cell image observation processing system 10, each of the plurality of observation processing apparatuses 1a-1 to 1a-n is provided with an observation information acquiring section 20, a tag acquiring section 11 and a storing section 12.

The other configuration is substantially the same as in the case of the cell information processing system 10 of FIG. 11.

In the cell observation information processing system 10 of FIG. 12 thus configured, at the stage of storing observation information on cells under culture into the archive section, in each of the observation processing apparatuses 1a-1 to 1a-2, the observation information acquiring section 20 acquires observation information in response to an acquisition order by a user at one time point, the tag acquiring section 11 acquires search tags for searching observation information acquired via the observation information acquiring section 20, and the storing section 12 assigns the search tags acquired by the tag acquiring section 11 to the observation information and stores the search-tagged observation information into the archive section 13 in the archive apparatus 1b via each network line.

The processing procedure at the stage of searching and retrieving desired observation information on cells from the archive section 13 and checking cell conditions is substantially the same as that in the cell information processing system 10 of FIG. 11.

According to the cell observation information processing system of FIG. 12, since the configuration is made so that a plurality of observation processing apparatuses 1a-1 to 1a-n (in FIG. 12, n = 2 for convenience' sake) acquire observation information and the observation information acquired by the respective observation processing apparatuses 1a-1 to 1a-n are managed in bulk, without being tied to the location of a particular observation processing apparatus 1a-n, a user can acquire observation information via an observation processing apparatus provided at a desired location and centrally record the search-tagged observation information acquired via the individual observation processing apparatuses into the single archive section 13, to enjoy much improved convenience.

Further, since the configuration is made so that the observation data acquired by a plurality of observation processing apparatuses 1a-1 to 1a-n are managed in bulk, if each of the observation processing apparatuses is configured to be small-sized and light-weighted as a mobile unit for exclusive use by a particular user, each user can acquire observation information on cells at any place, to enjoy much improved convenience.

The other configuration and function and effect are substantially the same as in the case of the modified example of FIG. 11.

In the embodiment mode above, the description is made on the configuration in which the tag acquiring section 11 acquires user identifying information, cell identifying information, date/time information for identifying the date/time when the observation information were acquired, information on user's work for cell maintenance, apparatus identifying information, activity data variation information and an image tag as separately independent tags. Alternatively, the tag acquiring section 11 may be configured to acquire any plurality of kinds of tags as linked together to form a single tag.

In the embodiment mode above, the description is made on the configuration in which the tag acquiring section 11 acquires, as search tags, tags inputted by a user in the search tag input screen such as "parent cell line" or "cells for experiment", a cell name and a passage number, and a user ID selected and inputted by the user in the user ID selection screen. Alternatively, the tag acquiring section 11 may be configured to have a function of displaying a screen for letting a user input search tags, for example a function of displaying a search tag input screen for letting a user input tags such as "parent cell line" or "cells for experiment", a cell name, and a passage number, or a function of displaying a user ID selection screen for letting a user input a user ID.

In the embodiment mode above, the description is made on the configuration in which the search criterion acquiring section 14 searches through the archive section 13 using a criterion inputted by a user in the search criterion designating screen and retrieves data containing search-tagged observation information to which search tags matched with the search criterion are assigned from the archive section 13. Alternatively, the search criterion acquiring section 14 may be configured to have a function of displaying a search criterion designating screen for letting a user input a search criterion.

According to the embodiment mode above, a captured image of a region about one local point (observation position) on a specimen containing cells is acquired. On the other hand, the system may be configured, as one modified example, to acquire, upon providing a plurality of cameras, a plurality of images by capturing images of a plurality of regions about respective local points (observation positions) at one time point using the plurality of cameras, and to treat the plurality of images acquired at one time point as one image group. In this case, one tag (regarding user ID, cell name etc.) is assigned not to each image but to one image group.

According to the embodiment mode above, the configuration is made so that tag acquisition and assignment is conducted via a manual input by a user; a manual input by a user is accepted via the display section such as a screen, and the tag acquiring section acquires the tags accepted via the display section and assigns the acquired tags to observation information on image etc. On the other hand, tag acquisition and assignment may be configured as shown in the following modified examples. Modified example 1: configuration in which the system automatically conducts tag acquisition or assignment

The tag acquiring section may be configured to preliminarily store tags that carry certain contents, and, on the occasion of tag assignment, to acquire and assign the tags stored in the system. For example, if the system is to be exclusively used by a certain user, the user ID of this user is preliminarily stored in the system.

According to the modified example 1, since the system automatically acquires and assigns tags, input work by user is reduced.

### Modified example 2 : configuration in which tag acquisition or assignment is conducted via alternative input mode by user

The tag acquiring sectionmay be configured so that tags are acquired and assigned via sound input, eye-gaze input or gesture input by a user.

For example in the case of voice input, the tag acquiring section converts voice information that has been vocally inputted by a user into text information using voice analyzing technique as disclosed in JP KOKAI No. 2012-252181, and assigns the text information as a tag to observation information on an image etc.

Also, for example in the case of eye-gaze input, the configuration may be made so that, upon correspondence information, in which moving directions of user's eye gaze are associated with certain letters, being stored in the system, the tag acquiring section detects directions of user's eye-gaze with an eye-gaze detecting section such as an eye-gaze detecting sensor, and acquires, as a tag, letters associated with the detected directions, on the basis of the detected directions of user's eye-gaze and the correspondence information.

Further, for example in the case of gesture input, the configuration may be made so that, upon correspondence information, in which moving directions of user's body parts such as limbs and trunk are associated with certain letters, being stored in the system, the tag acquiring section detects moving directions of user's body parts with a gesture detecting section such as a gesture detecting sensor, and acquires, as a tag, letters associated with the detected directions, on the basis of the detected moving directions of user's body parts and the correspondence information.

In this modified example, while examples of alternative input mode by user such as voice input and eye-gaze input are given, input mode by user is not limited to these. For example, the configuration may be made so that tags are acquired an assigned via input by brain waves.

In this way, according to the several embodiment modes of the present invention, it is possible to provide a cell observation information processing system, a cell observation information processing method, a cell observation information processing program, an archive section provided for the cell observation information processing system, and an apparatus provided for the cell observation information processing system, each of which enables users to efficiently conduct condition management of cells used for research of living object.

Alternatively, according to the several embodiment modes of the present invention, it is possible to provide a cell observation information processing system, a cell observation information processing method, a cell observation information processing program, an archive section provided for the cell observation information processing system, and an apparatus provided for the cell observation information processing system, each of which enables users to conduct, in a short time, condition management of cells used for research of living object.

Alternatively, according to the several embodiment modes of the present invention, it is possible to provide a cell observation information processing system, a cell observation information processing method, a cell observation information processing program, an archive section provided for the cell observation information processing system, and an apparatus provided for the cell observation information processing system, each of which enables users to accurately conduct condition management of cells used for research of living body.

Alternatively, according to the several embodiment modes of the present invention, it is possible to provide a cell observation information processing system, a cell observation information processing method, a cell observation information processing program, an archive section provided for the cell observation information processing system, and an apparatus provided for the cell observation information processing system, each of which enables users to rapidly understand cause of failure of experiments.

Alternatively, according to the several embodiment modes of the present invention, it is possible to provide a cell observation information processing system, a cell observation information processing method, a cell observation information processing program, an archive section provided for the cell observation information processing system, and an apparatus provided for the cell observation information processing system, each of which enables users to enhance success rate of experiments.

While the above explanation is made on the embodiment mode and its modified examples of the present invention, the present invention is not limited to the exact configuration as described in the embodiment mode and its modified examples. At the stage of reduction into practice, components can be embodied as being modified within the scope of not changing the essential concept of the invention. In addition, by appropriately combining a plurality of components described in the embodiment mode and its modified examples together, various inventions can be introduced. For example, some components may be removed from all of the components described in the embodiment mode and its modified examples or components described in different embodiment mode and its modified examples can be appropriately combined together. In this way, various modifications and applications are possible within the scope of not changing the essential concept of the invention.

### INDUSTRIAL APPLICABILITY

The cell observation information processing system, the cell observation information processing method, the cell observation information processing program, the archive section provided for the cell observation information processing system, and the apparatus provided for the cell observation information processing system are useful, other than in the fields where there is a demand for condition management of cells used for research of living object upon use of observation information on the cells acquired by an observation information acquiring apparatus such as a microscope, in fields where there is a demand for condition management of living object that changes as time passes.

### DESCRIPTION OF THE REFERENCE SYMBOLS

- 1: cell observation information processing installation
- 1a, 1a-1, 1a-2: observation processing apparatus
- 1b: archive processing apparatus
- 1c: search processing apparatus
- 10: cell observation information processing system
- 11: tag acquiring section
- 12: storing section
- 13: archive section
- 14: search criterion acquiring section
- 15: retrieving section
- 20: observation information acquiring section

## Claims

1. A cell observation information processing system comprising:
a tag acquiring section that acquires, as search tags for searching for observation information that has been acquired via an observation information acquiring section in response to an acquisition order at one time point to carry a cell observation result including at least one of a cell image, activity data indicating cell activity by at least one of a number of cells, a cell confluency, a morphology, and a survival rate, and an observation title, at least one of user identifying information for identifying a user who conducts cell observation, cell identifying information for identifying cells under observation, date and time information for identifying a date and time when the observation information was acquired, information on user's work for cell maintenance, apparatus identifying information for identifying an apparatus used in acquiring the observation information, activity data variation information, and an image tag for indicating the cell image;
a storing section that assigns the search tags acquired by the tag acquiring section to each set of the observation information, which has been acquired in response to an acquisition order at each time point, and that stores search-tagged observation information, to which the search tags have been assigned, into an archive section;
a search criterion acquiring section that acquires, as a search criterion, information containing, among the search tags assigned to the search-tagged observation information, at least one of the user identifying information, the cell identifying information, the information on user's work for cell maintenance, the apparatus identifying information, the activity data variation information, and the image tag; and
a retrieving section that searches through the archive section using the search criterion acquired by the search criterion acquiring section and retrieves data containing search-tagged observation information to which search tags that are matched with the search criterion have been assigned.

2. The cell observation information processing system according to claim 1,
**characterized in that**:
the archive section accommodates respective sets of the search-tagged observation information to which search tags adapted for cell observation by a plurality of users are assigned, and
the search criterion acquiring section acquires, as the search criterion, information containing the user identifying information.

3. The cell observation information processing system according to claim 2,
**characterized in that**:
the archive section accommodates respective sets of the search-tagged observation information to which search tags adapted for cell observation directed to a plurality of cells by a plurality of users are assigned, and
the search criterion acquiring section acquires, as the search criterion, information containing the user identifying information and the cell identifying information.

4. The cell observation information processing system according to claim 3,
**characterized in that**:
the cell identifying information contains at least one of a cell name, a cell kind, cell level information for distinguishing whether cells are in a parent cell line to be cultured or cells for experiment separated and cut out from the parent cell line for subculture, and a passage number of cells, and
the search criterion acquiring section acquires, as the search criterion, information containing the user identifying information and at least one of the cell name, the cell kind, the cell level information and the passage number.

5. The cell observation information processing system according to claim 4,
**characterized in that**:
the search criterion acquiring section acquires, as the search criterion, information containing the user identifying information and at least one of the cell name and the cell kind.

6. The cell observation information processing system according to claim 5,
**characterized in that**:
the search criterion acquiring section acquires, as the search criterion, information containing the user identifying information, at least one of the cell name and the cell kind, and the cell level information.

7. The cell observation information processing system according to claim 6,
**characterized in that**:
the search criterion acquiring section acquires, as the search criterion, information containing the user identifying information, at least one of the cell name and the cell kind, the cell level information, and the passage number.

8. The cell observation information processing system according to claim 3,
**characterized in that**:
the search criterion acquiring section acquires, as the search criterion, information containing the user identifying information, the cell identifying information and the activity data variation information.

9. The cell observation information processing system according to claim 2,
**characterized in that**:
the archive section accommodates each set of the search-tagged observation information to which search tags adapted for cell observation by a plurality of users with a plurality of types of work for cell maintenance are assigned, and
the search criterion acquiring section acquires, as the search criterion, information containing the user identifying information and the information on user's work for cell maintenance.

10. The cell observation information processing system according to claim 1,
**characterized in that**:
the archive section accommodates each set of the search-tagged observation information to which search tags adapted for cell observation directed to a plurality of types of cells are assigned, and
the search criterion acquiring section acquires, as the search criterion, information containing the cell identifying information.

11. The cell observation information processing system according to claim 10,
**characterized in that**:
the search criterion acquiring section acquires, as the search criterion, information containing the cell identifying information and the activity data variation information.

12. The cell observation information processing system according to claim 1,
**characterized in that**:
the user identifying information is identifying information for identifying a very person concerned who conducts input of the cell observation result or an acting person who conducts cell observation and input of the cell observation result in place of the very person concerned.

13. The cell observation information processing system according to claim 1,
**characterized in that**:
the cell identifying information contains at least one of a cell name, a cell kind, cell level information for distinguishing whether cells are in a parent cell line to be cultured or cells for experiment separated and cut out from the parent cell line for subculture, and a passage number of cells.

14. The cell observation information processing system according to claim 13,
**characterized in that**:
the search criterion acquiring section acquires, as the search criterion, information containing at least one of the cell name and the cell kind, and at least one of the cell level information and the passage number.

15. The cell observation information processing system according to claim 1,
**characterized in that**:
the information on user's work for cell maintenance contains at least one of a work detail, a dilution rate, a container's type or size, and a container address where the cells are stored.

16. The cell observation information processing system according to claim 1,
**characterized in that**:
the activity data variation information is an amount of variation between respective activity data at a plurality of time points.

17. The cell observation information processing system according to claim 1,
**characterized in that**:
the observation information is constructed of information that carries a plurality of observation results corresponding to image capture at one time point directed to a plurality of observation positions on a specimen containing the cells.

18. The cell observation information processing system according to claim 17,
**characterized in that**:
the information carrying the plurality of observation results contains a plurality of cell images.

19. The cell observation information processing system according to claim 17,
**characterized in that**:
the information carrying the plurality of observation results contains a value calculated out on a basis of the plurality of cell images.

20. The cell observation information processing system according to claim 17,
**characterized in that**:
the information carrying the plurality of observation results contains a plurality of cell images and a value calculated out on a basis of the plurality of cell images.

21. The cell observation information processing system according to claim 17,
**characterized in that**:
the information carrying the plurality of observation results contains a plurality of cell images, and
the search tags include a value calculated out on a basis of the plurality of cell images.

22. The cell observation information processing system according to claim 17,
**characterized in that**:
the information carrying the plurality of observation results contains a plurality of cell images, and
the search tags include an image tag for indicating the plurality of cell images.

23. The cell observation information processing system according to claim 17,
**characterized in that**:
the information carrying the plurality of observation results contains a value calculated out on a basis of a plurality of cell images, and
the search tags include the value calculated out on a basis of the plurality of cell images.

24. The cell observation information processing system according to claim 17,
**characterized in that**:
the information carrying the plurality of observation results contains a plurality of cell images, and
the search tags include an image tag for indicating the plurality of cell images and a value calculated out on a basis of the plurality of cell images.

25. A cell observation information processing method comprising:
a tag acquiring step for acquiring, as search tags for searching for observation information that has been acquired in response to an acquisition order at one time point to carry a cell observation result including at least one of a cell image, activity data indicating cell activity by at least one of a number of cells, a cell confluency, a morphology, and a survival rate, and an observation title, at least one of user identifying information for identifying a user who conducts cell observation, cell identifying information for identifying cells under observation, date and time information for identifying a date and time when the observation information was acquired, information on user's work for cell maintenance, apparatus identifying information for identifying an apparatus used in acquiring the observation information, activity data variation information, and an image tag for indicating the cell image;
a storing step for assigning the search tags acquired at the tag acquiring step to the observation information, which has been acquired in response to an acquisition order at each time point, and for storing search-tagged observation information, to which the search tags have been assigned, in an archive section;
a search criterion acquiring step for acquiring, as a search criterion, information containing, among the search tags assigned to the search-tagged observation information, at least one of the user identifying information, the cell identifying information, the information on user's work for cell maintenance, the apparatus identifying information, the activity data variation information, and the image tag; and
a retrieving step for searching through the archive section using the search criterion acquired at the search criterion acquiring step and for retrieving data containing search-tagged observation information to which search tags that are matched with the search criterion have been assigned.

26. A cell observation information processing program, **characterized by** making a computer function as:
a tag acquiring means that acquires, as search tags for searching for observation information that has been acquired in response to an acquisition order at one time point to carry a cell observation result including at least one of a cell image, activity data indicating cell activity by at least one of a number of cells, a cell confluency, a morphology, and a survival rate, and an observation title, at least one of user identifying information for identifying a user who conducts cell observation, cell identifying information for identifying cells under observation, date and time information for identifying a date and time when the observation information was acquired, information on user's work for cell maintenance, apparatus identifying information for identifying an apparatus used in acquiring the observation information, activity data variation information, and an image tag for indicating the cell image;
a storing means that assigns the search tags acquired by the tag acquiring means to the observation information, which has been acquired in response to an acquisition order at each time point, and that stores search-tagged observation information, to which the search tags have been assigned, in an archive section;
a search criterion acquiring means that acquires, as a search criterion, information containing, among the search tags assigned to the search-tagged observation information, at least one of the user identifying information, the cell identifying information, the information on user's work for cell maintenance, the apparatus identifying information, the activity data variation information, and the image tag; and
a retrieving means that searches through the archive section using the search criterion acquired by the search criterion acquiring means and retrieves data containing search-tagged observation information to which search tags that are matched with the search criterion have been assigned.

27. The archive section provided for the cell observation information processing system according to claim 1,
**characterized in that**:
the archive section has one or more records of the search-tagged observation information,
each of the records is created in response to image capture of cells at one time point, and
the observation information corresponding to the image capture of cells at one time point and the search tags for searching for the observation information are interrelated.

28. A plurality of apparatuses provided for the cell observation information processing system according to claim 1,
**characterized in that**:
the tag acquiring section, the storing section, the search criterion acquiring section and the retrieving section are separately arranged in the plurality of the apparatuses.

29. The cell observation information processing system according to claim 1,
further comprising a display section that displays data containing the search-tagged observation information retrieved by the retrieving section.
